# EUROPEAN PATENT APPLICATION

(11) **EP 3 579 579 A1**
(43) Date of publication of application: **11.12.2019**
(21) Application number: 18176351.7
(22) Date of filing: 06.06.2018
(51) Int. Cl.: H04R 25/00

(54) **SECURING A UNIFORM RESOURCE INDICATOR FOR COMMUNICATING BETWEEN A HEARING CARE PROFESSIONAL AND A HEARING DEVICE USER**

(71) Applicant: Sonova AG, 8712 Stäfa (CH)
(72) Inventor: Schneider, Phillipp, 8049 Zürich (CH); Tsitovich, Aliaksei, 8712 Stäfa (CH); Wojcieszyn, Filip, 8712 Staefa (CH)

(57) **Abstract**

The disclosed technology generally relates to securing electronic communication between a hearing care professional (HCP) and a hearing device user. The disclosed technology relates to digitally signing a URI with a hearing device, transmitting the digitally signed URI to an electronic device accessible by a user, and verifying the digitally signed URI.

## Description

### TECHNICAL FIELD

The disclosed technology generally relates to securing electronic communication between a hearing care professional (HCP) and a hearing device user. For example, the disclosed technology relates to using a hearing device and a private key stored in the hearing device to digitally sign the URI. After the digitally signed URI is verified, it can be used for secure communication or secure information access.

### BACKGROUND

Hearing device users and HCPs want to communicate over the Internet securely. Communicating includes accessing, reading, writing, or storing information on the cloud, on a computer, on a network, or sending or receiving electronic communication at a hearing device. For example, an HCP may want to establish a secure connection with a user and his or her hearing device to conduct a fitting session for the hearing device. A fitting session is an appointment where an HCP communicates with a hearing device user regarding hearing device settings.

Currently, passwords or usernames protect communication, but this is not fully secure because passwords and usernames can be stolen. For example, patent application WO 2016/078710 discloses a system for granting access rights to data for a hearing aid user by creating a user account on a remote server accessible over the Internet. However, hackers can steal passwords or usernames or circumvent passwords and usernames (to send phishing links or spurious communication e.g., guessing a password by a brute force to and generating a false link or message).

Other techniques have been used to secure communication with a hearing device user. For example, Pedersen et al. (US 9,608,807) discloses a hearing device system for communicating between a hearing device and a computer. Pedersen's hearing device includes a processing unit that is configured to receive a session request for a session, obtain and store a session key and encrypt the session key based on a hearing device key. Based on the encrypted session key, the hearing device can communicate with the computer. However, Pedersen's disclosed technology is still subject to attacks, spoofing, or other methods of improper access and not fully secure.

Therefore, there still exists a need to improve the security of internet communications to prevent unsecure or untrusted communication with respect to hearing devices. Additionally, because hearing device data is considered sensitive information (e.g., medical or confidential), companies and user should securely communicate hearing device data.

### SUMMARY

This summary provides concepts of the disclosed technology in a simplified form that are further described below in the Detailed Description.

The disclosed technology includes a method to communicate using a hearing device, wherein the method includes a hearing device configured to digitally sign a uniform resource indicator (URI) and a fitting station configured to communicate with the hearing device. The method can comprise operations for generating a URI; transmitting the URI to the hearing device; signing the URI to generate a digitally signed URI; transmitting the digitally signed URI from the fitting station to an electronic device; verifying the digitally signed URI at the electronic device; and based on verifying the digitally signed URI, using the digitally signed URI to establish a session between the fitting station and the hearing device or using the digitally signed URI to access a network or computer address. Transmitting the URI to the hearing device can be performed using a Bluetooth™ communication protocol or other wireless protocol.

The method can also include using a private key stored in a memory for the hearing device, where the private key is associated with a public key and the electronic device has access to the public key, is configured to receive access to the public key, or is configured to receive the public key. The private key and the public key can be an asymmetric key pair. The method can include distributing the public key over a network, where verifying the digitally signed URI includes using the distributed public key at the electronic device to verify the digitally URI. Although a hearing device manufacturer can install the private key or public key on the hearing device during the production of the hearing device, in some implementations, the hearing device can generate the private key or the public key.

The digitally signed URI can be configured to expire, e.g., within 2 weeks or after 1 year. The digitally signed URI can be associated with a fitting session, fitting data, a hearing device, a user of the hearing device, or an invitation from an HCP.

The electronic device can be a mobile device configured to use an application to use the digitally signed URI. For example, the mobile device can use a mobile application to send a request to the hearing device to access the URI (stored on the hearing device) or the mobile device can receive the URI (e.g., via user input or from an email) and then verify whether the URL has been digitally signed with a public key.

The disclosed technology can include a hearing device configured to perform the method. The hearing device can be a hearing aid, earbud, cochlear implant, or cochlear device. Also, the hearing device can be binaurally paired with another hearing device. The hearing device can also include a memory storing instructions to sign a URI or use a private key to sign a URI. The hearing device memory can also include a hashing function or a security algorithm configured to perform hashing algorithms or operations.

In some implementations, the method is stored on a computer-readable medium, where the method is a computer program. A processor physically or electronically coupled to the hearing device can access the computer program and execute the instructions.

### BRIEF DESCRIPTION OF THE DRAWINGS

The accompanying figures are some implementations of the disclosed technology.
Figure 1A illustrates a distance communication environment with hearing devices, a fitting station, a network, and an electronic device in accordance with some implementations of the disclosed technology.
Figure 1B illustrates a more detailed schematic view of the memory for the hearing device from Figure 1A in accordance with some implementations of the disclosed technology.
Figure 2 illustrates a schematic block diagram for digitally signing a URI that can be used for exchanging or accessing information in accordance with some implementations of the disclosed technology.
Figure 3 is a block flow diagram illustrating a process for generating a URI, digitally signing the URI, and using the digitally signed URI for secure communication in accordance with some implementations of the disclosed technology.

The figures are not drawn to scale and have various viewpoints and perspectives. Some components or operations shown in the figures may be separated into different blocks or combined into a single block for the purposes of discussion. The disclosed technology is intended to cover all modifications, equivalents, and alternatives falling within the scope of the appended claims.

### DETAILED DESCRIPTION

The disclosed technology generally relates to securing electronic communication between a HCP, a hearing device, and/or a user of the hearing device. The disclosed technology relates to digitally signing a URI, transmitting the digitally signed URI to an electronic device that a user has access to, and verifying the digitally signed URI. After the digitally signed URI is verified, a user can securely access information associated with the URI or establish a secure connection with another person or computer (e.g., an HCP or fitting station associated with the HCP). The URI is a generic string of characters designed for unambiguous identification of a resource, e.g., a URI to a database, network, web address, computer address, resource, fitting data, calendar invite, or fitting session.

In some implementations, the disclosed technology includes a hearing device that has a memory, where the memory stores a private or public key (also referred to as a "private key and public key pair"). The private key and public key pair can be an asymmetrical key pair because the private and public keys are used for asymmetric cryptography. Asymmetrical also means if the private key digitally signs a message, then the public key can be used to determine that the private key digitally signed it. The public key can be shared with everyone, where the private key is kept secret.

In some implementations, the hearing device stores only the private key. In other implementations, the hearing device stores the private key and the public key. The hearing device manufacturer can install the private key or the public key or both on the hearing device. In some implementations, the hearing device has its own key generation algorithms, which can be used to generate only a private key, only a public key, or both a private and public key.

To create a digital signature, a hearing device can use signing software with a one-way hash function and a private key. Here, the private key, URI, and the one-way hash function are used to encrypt the hash, returning a value that is unique to the hashed data (e.g., a signed URI). The encrypted hash, along with other information such as the hashing algorithm, forms the digital signature. Any change in the data, even to a single bit, results in a different hash value. This attribute enables others to validate the integrity of the data by using the public key to decrypt the hash. If the decrypted hash matches a second computed hash of the same data, it proves that the data has not changed since it was signed. If the two hashes do not match, the data has either been tampered with in some way (indicating a failure of integrity) or the signature was created with a private key that does not correspond to the public key presented by the signer (indicating a failure of authentication). As an example, the one-way hash function can be used with the private key and URI to generate a one-way hashed output. The hashed output can be a 64-bit one-way hashed string, where the 64-bit string includes the URI and characters corresponding to an output of the hash function from the private key.

When a user receives a digitally signed URI, the user can use the public key to determine if the private key signed the URI. If the URI was digitally signed, the URI has a digital signature that indicates the sender of the URI had the private key and this can be verified by anyone who access to the public key. With the disclosed technology, it is computationally difficult for anyone who does not know the private key to deduce it from the public key or any number of signatures or to find a valid signature on any message for which a signature has not been used. Thus, the authenticity of a URI can be demonstrated by the digital signature, provided the trusted authority of the private key keeps the private key secret. And in this case, the trusted authority is the hearing device because the HCP and the user agree to trust the hearing device. For example, the HCP or hearing device manufacturer can install the private key in a hearing device, and the HCP or hearing device manufacturer can distribute the public key.

In some implementations, the digitally signed URI can include additional security measures. The digitally signed URI can be set to expire after a period. For example, the hearing device can sign the URI such that the digitally signed URI is only valid for 2 weeks or 2 months. Also, the hearing device manufacture can control the distribution of the public key. For example, the hearing device manufacture can email the public key only to parties that have registered a hearing device.

The disclosed technology can assist in setting up a fitting session or communicating between an HCP and hearing device user. For example, an HCP can receive new hearing devices directly at his or her office with a pre-installed private key and public key. If the private key and public key are not preinstalled, the hearing device can use the encryption software to generate a private key and public key pair. If the HCP wants to send a URI to a hearing device user, e.g., to set up a fitting session or distance support session, the HCP can generate a URI and send it to the hearing device such that the hearing device can sign the URI.

Once the hearing device digitally signs the URI, the HCP can send the digitally signed URI to a user. The user can connect the hearing device with their electronic device such as a mobile device, and the hearing device can share the public key associated with the private key with the mobile device. Then, once the user receives the URI, the electronic device or a server can use the public key to verify whether the digital signature on the URI is authentic. If the signature is authentic, then the user can connect to the HCP using a secure URI, and if it is not authentic, the electronic device can prevent the user from connecting or just warn the user that the URI may not be authentic.

In some implementations, the disclosed technology has at least one technical solution to securing electronic communication between an HCP and a user or between a hearing device user and some data. One technical solution is a tamper resistant URI. The URI is tamper resistant because it signed with a private key and the private key and an HCP or hearing device manufacturer securely possessed the private key and the digital signature was determined with a hash function, which can be used to indicate if the URI was modified. The chain of authority between the hearing device manufacture, the HCP, and/or the hearing device user is not broken. Also, the hearing device user and the HCP agree to trust the hearing device. The private key can also be configured to not leave the hearing device, and the signing operation can be configured to only happen in the hearing device, which further increases the security of the private key. Another technical solution is that the URI can include security measures such as generating different and new URIs for different communication or causing the URI to expire after a period. Because the URI is tamper resistant and includes security, the disclosed technology reduces the risk of spoofed links or falsely generated links because the URI is digitally signed.

Also, the disclosed technology reduces the need for a separate or additional trusted authority to sign the URI. For example, the HCP and the hearing device can work together solely to verify or sign a URI. It is not necessary to have a separate trusted authority or another service for authentication or verification. Reducing the number of parties involved reduces processing time, increases security (e.g., more parties may create more security risks), reduces costs, and allows others (e.g., hearing care providers) to use this solution as a "plug-and-play" solution without requiring a separate database or login portal. In some implementations, other login portals, trusted authorities, or databases can be added.

A digital signature also makes it difficult for the signing party to deny having signed something. If a signing party denies a valid digital signature, their private key has either been compromised, or they are being untruthful.

The disclosure technology adds functionally to the hearing device such as a private key storage, public key storage, encryption, and the ability to sign URIs to improve security in the computer environment.

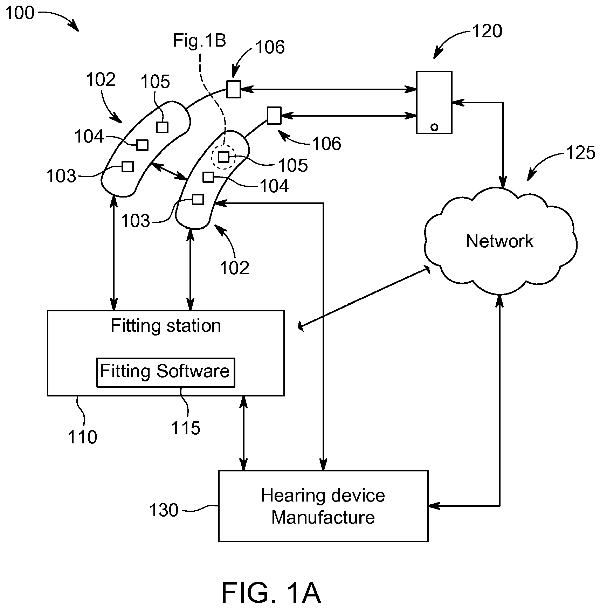

Moving to the Figures, Figure 1A illustrates is a communication environment 100 with hearing devices, a fitting station, a network, and a mobile device. The communication environment 100 includes a hearing device 102 (also referred to in the plural as "the hearing devices 102"), a fitting station 110 (e.g., a computer that a HCP can use to fit hearing devices 102) with fitting software 115, and a network 125 used for communicating between a device and the Internet or a private network. Also, shown for reference in the communication environment 100 is the hearing device manufacturer 130 of the hearing devices 102. The hearing device manufacturer 130 produces the hearing devices 102 and can install hardware or software on the hearing devices 102.

An HCP can use the communication environment 100 to communicate with a hearing device user, where the hearing device user has an electronic device 120 such as a mobile device or personal computer. Because the hearing devices 102 have a limited user interface (e.g., no graphical user interface), the electronic device 120 is used to facilitate interactions between the hearing devices 102 and the user of the hearing devices 102. A distance fitting session can be conducted when the hearing device user is at home or any location that has Internet access. For example, the HCP and hearing device user can setup a video conferencing session of Voice-over Internet Protocol (VoIP) session to discuss issues related to hearing devices 102. Although fitting sessions are disclosed in this disclosure, the communication environment 100 can also be used for general electronic communication between the HCP and the hearing device user.

Regarding the hearing devices 102, the hearing devices 102 are devices that provide audio to a user wearing the hearing device. The hearing devices 102 can do this individually or as a combination (e.g., stereo audio or modify audio different for each ear). Some example hearing devices include a hearing aid, headphones, earphones, assisted listening devices, or any combination thereof; and hearing devices include both prescription devices and non-prescription devices configured to be worn on a human head. A hearing aid is a device that provides amplification or attenuation of audio signals to compensate for hearing loss or attenuation functionalities; some example hearing aids include a Behind-the-Ear (BTE), Receiver-in-the-Canal RIC, In-the-Ear (ITE), Completely-in-the-Canal (CIC), or Invisible-in-the-Canal (IIC) hearing aids. A hearing device can also include a cochlear implant, where the cochlear implant (e.g., including an implant part and a device part) can communicate with a hearing device. In some implementations, the hearing devices 102 refer to a combination of the same devices (e.g., earbuds or identical hearing aids) or different devices (e.g., a hearing aid and a cochlear device or a CIC or RIC). Components of the hearing devices 102 are described in more detail below.

The hearing devices 102 include a processor 103, an antenna 104, a memory 105, and a loudspeaker 106 (also referred to as a "receiver") (collectively referred to as "hearing device components"). Each of the hearing device 102 components are described below.

The processor 103 processes information for the hearing device 102 and the processor 103 is configured to control the hearing device 102. The processor 103 can include special-purpose hardware such as application specific integration circuits (ASICS), programmable logic devices (PLDs), field-programmable gate arrays (FPGAs), programmable circuitry (e.g., one or more microprocessors microcontrollers), Digital Signal Processor (DSP), appropriately programmed with software and/or computer code, or a combination of special purpose hardware and programmable circuitry. The processor 103 can perform hash functions or hash computations.

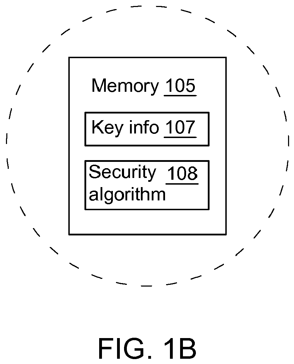

The processor 103 is physically and electronically coupled to memory 105 such as volatile memory, nonvolatile memory, or dynamic memory. In some implementations, the memory 105 can store a private key, libraries of code for signing a URI, encryption algorithms (see Figure 1B), or a public key. For example, a hearing device manufacturer 130 can store a private key in the memory 105 such that the hearing device 102 is the only device that can read or use the private key (e.g., restricted memory access). The hearing device manufacturer 130 can also install libraries of code that can be read or used by the processor 103 to sign or read URIs sent to the hearing devices 102, which is also referred to as signing software.

In some implementations, the hearing device 102 can be configured to use stored encryption algorithms to generate its own private key, public key pair, or private and public key pair. The memory 105 also stores data, information, or algorithms for operating the hearing devices 102 to improve or provide sound to a user. In some implementations, the memory 105 is physically divided into two separate sections, where one section stores the private key or algorithm for generating a private or public key, and another section stores data related to providing hearing.

As shown by the double-headed arrow between the hearing devices 102 in Figure 1, the hearing devices 102 can also communicate with each other (e.g., binaural communication). For example, binaural communication can include exchanging information related to improving a user's hearing such as beamforming information or volume or gain control. In some implementations, binaural communication occurs on a different frequency or with a different protocol compared to communicating with external devices (e.g., devices besides the hearing devices 102). Each component of the hearing devices 102 is described below in more detail.

To wireless communicate with other devices, the hearing devices 102 use the antenna 104. The antenna 104 can be a single antenna or an array of antennas. The antenna 104 is configured to transmit and receive wireless communication signals in frequency bands (e.g., in the 2.4 GHz frequency band for Bluetooth Classic™, Bluetooth Low Energy™, or a range of frequencies from 1 to 6 GHz). As shown in Figure 1, the antenna 104 can be completely inside the housing (e.g., plastic housing). However, in some implementations, the antenna 104 can be partially or completely outside of the housing depending on desired transmission and propagation properties. For hearing devices, it is generally preferred to protect the hearing device components from moisture or environmental conditions by having all the hearing device components inside the housing. Accordingly, the housing can be composed of material that enables the transmission of wireless communication signals, but also reduces (e.g., prevents) the entrance of moisture (e.g., plastic). Further, in some implementations, the hearing device 102 can have more than 1 antenna where each antenna is configured to operate on a different frequency or for a different purpose (e.g., Wi-Fi or Bluetooth™).

The hearing devices 102 can include additional components. For example, if the hearing devices 102 are configured for wireless inductive charging, the hearing devices 102 can include a coil to receive a magnetic field. The coil can vary in size, shape and construction depending on the size of the hearing devices 102 and the designed power to be received by the hearing device 102. The hearing devices 102 can include additional components. For example, if the hearing devices 102 are configured for wireless inductive charging, the hearing devices can include a coil to receive a magnetic field. The coil can vary in size, shape and construction depending on the size of the hearing devices 102 and the designed power to be received by the hearing device. As another example, the hearing devices 102 can have a microphone or microphones. The microphone or microphones can be used for own-voice detection or receiving sound related to the hearing device environment.

With further reference to Figure 1, the electronic device 120 can be a mobile device, smart phone, tablet computer, laptop computer, desktop computer, a media device, a gaming device, virtual or augmented reality headset, vehicle-based computer, wearable computing device, or portable electronic device. In some implementations, the electronic device 120 includes software or an application that controls or communicates with the hearing devices 102. In some implementations, the hearing device 102 or the hearing devices 102 can communicate with the electronic device 120 using, e.g., Bluetooth™ or ZigBee™, or any proprietary protocol where signals are propagated between from the antenna 104 at the hearing devices 102 to an antenna at the electronic device 120 (e.g., bidirectional, or unidirectional communication). For example, the electronic device 120 can be a smartphone with an application (e.g., mobile app) or software program that provides a user interface for the hearing device user to communicate with the user.

The fitting station 110 can be a computer or a terminal connected to a server or cloud computing service. For example, the fitting station 110 can be a computer in a HCP's office (e.g., a home office), where the fitting station 110 is used to fit hearing devices 102. The fitting station 110 is configured to communicate with the hearing device 102 or the hearing devices 102, e.g., via Bluetooth™ or another wireless communication protocol. The fitting station 110 can be portable (e.g., laptop) or fixed in a location (e.g., a desktop at the HCP's office). The fitting station 110 is configured to generate a URI, where the URI can be read or used by a computer.

The fitting station 110 can include fitting software 115. The fitting software 115 is a set of instructions that can program or adjust the hearing device 102 or the hearing devices 102. The fitting software 115 can incorporate user experience values or user experience settings such as how a user feels or how a user perceived a sound (e.g., dog bark, conversation, high pitched noise) or a sound environment (e.g., movie theater, quite room). The fitting software 115 can personalize settings to accommodate individual user preferences and listening needs. The fitting software 115 can adjust hearing device settings for speech, own voice, overall loudness, and individual phonemes for speech recognition and intelligibility. Adjustments affect gain levels and frequencies which are relevant to enhance the audibility of the selected stimuli. The fitting software 115 can also generate visual displays or graphical user interfaces for the hearing device user and the HCP. The visual displays can include information about the hearing device 102 or the hearing devices 102.

The network 125 enables the hearing devices 102 to send and receive information from the Internet via the electronic device 120. For example, the network 125 can be a Wi-Fi™ network or a networking implementing an Institute of Electrical and Electronics Engineers (IEEE) 802.11 standard and the electronic device 120 can connect with network 125 to receive and send information, and the electronic device 120 can also send and receive information to and from the hearing devices 102 (or both hearing devices 102). In another example, the hearing devices 102 can communicate directly with the network 125, e.g., if network 125 is a Bluetooth™ network or 802.11 IEEE standard wireless communication network, where the hearing devices 102 are configured to communicate with the network 125.

Also, the network 125 can be a single network, multiple networks, or multiple heterogeneous networks, such as one or more border networks, voice networks, broadband networks, service provider networks, Internet Service Provider (ISP) networks, and/or Public Switched Telephone Networks (PSTNs), interconnected via gateways operable to facilitate communications between and among the various networks. The network 125 can include communication networks such as a Global System for Mobile (GSM) mobile communications network, a code/time division multiple access (CDMA/TDMA) mobile communications network, a 3^{rd}, 4^{th} or 5^{th} generation (3G/4G/5G) mobile communications network (e.g., General Packet Radio Service (GPRS/EGPRS)), Enhanced Data rates for GSM Evolution (EDGE), Universal Mobile Telecommunications System (UMTS), or Long Term Evolution (LTE) network), or other communications network such as a Wireless Local Area Network (WLAN).

Figure 1B illustrates a more detailed schematic view of the memory 105 inside of the hearing device 102 from Figure 1A. The memory 105 can include key information 107 and security algorithm 108. The key information 107 can include a private key, public key, or combination of private and public keys. The processor 103 can read or access the private key or public key by accessing the memory 105. In some implementations, the memory 105 is configured to prevent the key information 107 from leaving the hearing device 102, e.g., all key related operations, signing operations, or security algorithms can only occur in the hearing device 102. In some implementations, the hearing device manufacturer 130 installs a private key and public key in the memory 105 when the hearing device is manufactured. In other implementations, the hearing device 102 is configured to generate its own keys or key pairs using the security algorithm 108. The hearing device 102 can use the algorithms stored in its memory 105 to ensure a digitally signed link was hardware encrypted by the hearing device.

The security algorithm 108 can includes classes, libraries, and other instructions for carrying out computer operations related to securing communication. For example, the security algorithm 108 can store a private key and public key generator. The security algorithm 108 can include a cryptographic hash function to generate a key pair or hash a URI, a Rivest-Shamir-Adleman (RSA) scheme, or a function to digitally sign or produce a digitally signed URI. In some implementations, to create a digital signature, the security algorithms include a signing software that creates a one-way hash of data. The private key, the URI, and an unsigned URI are put into the one-way hash function and the hash function returns a value that is unique to the hashed data (e.g., a signed URI based on the private key and unsigned URI). Accordingly, the encrypted hash, along with other information such as the hashing algorithm, forms the digital signature. Any change in the data, even to a single bit, results in a different hash value. This attribute enables others to validate the integrity of the data by using the public key to decrypt the hash. If the decrypted hash matches a second computed hash of the same data, it proves that the data has not changed since it was signed. If the two hashes do not match, the data has either been tampered with in some way (indicating a failure of integrity) or the signature was created with a private key that does not correspond to the public key presented by the signer (indicating a failure of authentication).

As an example, the one-way hash function can be used with the private key and URI to generate a one-way hashed output. The hashed output can be a 64-bit one-way hashed string, where the 64-bit string includes the URI and characters corresponding to an output of the hash function from the private key. In some implementations, the output of the hash function can also include information about which hashing function or security algorithm was used to digitally sign the URI. Then, when a user receives the digitally signed URI, the user can look up the hashing function and use the public key to determine whether the URI was digitally signed with the private key. In other implementations, the digitally signature does not include the hash function or security algorithm that was used to digitally sign URI. In such implementations, the hearing device manufacturer can email or send the hash function to the user, and then the use can use the known hash function and the public key to determine if the private key digitally signed the URI.

The security algorithm 108 can also include instructions for signing a URI with a key or distributing a public key. In some implementations, the private key can be defined as a "the signing key" and public key is the "verification key". More generally, the ability of the hearing device to locally use a private key to sign URI improves security of communications because a digitally signed signature adds an additional layer of security.

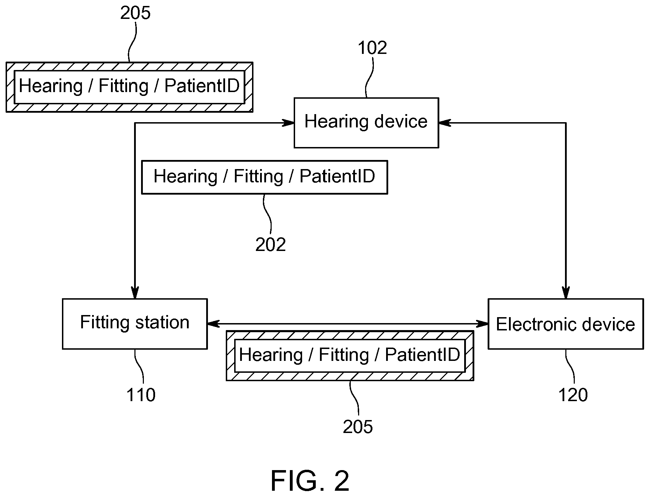

Figure 2 illustrates schematically block diagram for securing a URI that can be used for exchanging information between an HCP and a user. Figure 2 schematically shows the fitting station 110 (Figure 1), the electronic device 120 (Figure 1), and the hearing device 102. The fitting station 110, the electronic device 120, and the hearing device 102 can communicate, for example, to exchange a URI 202 and a digitally signed URI 205. The URI 202 can be referred to as an "unsigned URI 202", which means that it has not been signed.

A URI 202 is a generic string of characters designed for unambiguous identification of resources. The URI 202 can be a "Globally Unique Identifier" (GUID), Universally Unique Identifier (UUID), or a Uniform Resource Locator (URL) (also referred to as "link"). In some implementations, the URI 202 is an integer number used to identify resources (e.g., file location, server address, or location on a computer network or in a computing device). An example of the text associated with the URI 202 is show in Figure 2, "/hearing/fitting/patient/ID". In some implementations, the URI 202 includes information associated with the hearing device identification (e.g., serial number, product number, or other number associated with the hearing device or the hearing devices), a fitting session identification (e.g., a number associated with a fitting session for the hearing devices or devices), or information deemed necessary by the HCP. In some implementations, the fitting station 110 (e.g., a computer for the HCP) generates the URI 202 and it is an unsigned URI. The fitting station can transmit the unsigned URI 202 to the hearing device 102 for digital signature.

The hearing device 102 digitally signs the URI 202 and then the URI 202 becomes a digitally signed URI 205. The hearing device 102 can transmit the digitally signed URI 205 to the fitting station 110. In other implementations, the hearing device 102 can also transmit the digitally signed URI 205 to the electronic device 120 via the Internet or a Bluetooth™ connection. The fitting station 110 can also send the digitally signed URI to an electronic device 120. For example, the fitting station can email a potential customer or customer with a URI for scheduling an appointment for a fitting session. The customer can receive the digitally signed URI 205 on his or her phone, at his or her computer, or on a mobile application. In some implementations, the fitting station 110 transmits the digitally signed URI 205 to the electronic device 120, where the electronic device 120 is a mobile device with an application and the application can process and provide the digitally signed URI 205 to the user or use the digitally signed URI 205. For example, a user can use his or her mobile device a hearing device mobile application to schedule an appointment with an HCP or contact the HCP based on the digitally signed URI 205. Because the fitting station, HCP, or the hearing device manufacturer can freely distribute the public key, a user or other electronic device can access the public key to read or use it.

In some implementations, an HCP can print the digitally signed URI and mail it to a user. For example, the HCP can send the digitally signed URI by post or by mail to user. In this implementation, the user can receive the digitally signed URI in the mail and use it to access information associated with the URI (e.g., by typing it into an internet web browser). In some implementations, the HCP sends the digitally signed URI as a piece of paper with a QR code, which a user can scan or read when he or she receives it in the mail. The QR code can include a link address or URI related to the fitting data.

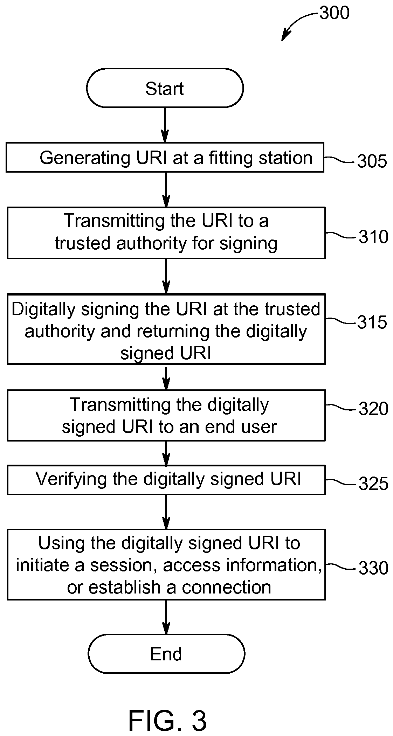

Figure 3 is a block process flow diagram for a process 300 for generating a URI, digitally signing the URI to secure the URI, transmitting the digitally signed URI to an electronic device, verifying the digitally signed URI at the electronic device or with a hearing device, and communicating securely using the digitally signed URI. The process 300 can begin when a HCP wants to communicate with a hearing device user or wants to set up a fitting session for the first time (or second time) with a hearing device user. The process 300 can be triggered to start automatically once the HCP requests to send an email, hearing device, or fitting session request to a hearing device user.

At generate URI operation 305, the fitting station 110 generates a URI. The URI can be a GUID, UUID, or another computer readable URI. The URI can provide a user, computer, or hearing device with a location or way to connect securely with another device or network. The URI can also be an address or link (e.g., URL) to a database or location for fitting data. In some implementations, the URI can be related to setting up a fitting session for a hearing device user. In some implementations, the HCP uses the URI to generate a location to start a distance fitting session, where the HCP is located remotely from the hearing device user (e.g., in another city, another building, or another country). In some implementations, the generated URI relates to patient's data or fitting data for a hearing device or two hearing devices.

At transmit URI operation 310, the fitting station 110 can transmit the URI to a hearing device 102. The fitting station 110 can transmit the URI using Wi-Fi or Bluetooth™ or another wireless communication protocol described in Figure 1. In some implementations, the transmit URI operation 310 occurs in an HCP's office and when the HCP has received a pair of hearing devices that the HCP intends to give or send to a user. Because the HCP received the hearing device at his or her office from a hearing manufacturer and the HCP is considered a trusted authority (as well as the hearing device manufacturer), the operation includes built in security measures to prevent others from generating false URIs because there is an unbroken chain of authority. The trusted authority is general defined as a party that all parties trust (e.g., the hearing device user and HCP agree to trust the hearing device).

At digitally signing URI operation 315, the hearing device 102 digitally signs the URI. By signing the URI, the URI is now secure because it is assumed that the hearing device is a trusted authority. Because the HCP received the hearing device from the hearing device manufacturer and the URI was signed in the presence of the HCP or under the control of the HCP, the assumption of security is likely a good assumption because the chain of title or chain of possession was not broken or interrupted.

Regarding the digitally signing URI operation 315, the signing operation can include using a private key. The private key can be stored in the memory 105 of the hearing device 102 and the memory 105 can be configured to only be accessible or usable in the hearing device 102. More specifically, the private key may be configured to never leave the hearing device 102 or configured such that only the hearing device 102 can perform the signing operation in the hearing device 102 because the private key is stored in a restricted memory. Also, using the hearing device itself for the authority, means a chain of command is expected and cannot be faked without the hearing device physically being present.

At transmitting digitally signed URI operation 320, the fitting station 110 can transmit the digitally signed URI to an end user. For example, the fitting station 110 can send an email to a user, where the email includes the digitally signed URI. The user can receive the digitally signed URI at his or her electronic device (e.g., using an email application or mobile application). In some implementations, an HCP can print the digitally signed URI and mail it to a user. For example, the HCP can send the digitally signed URI by post or by mail to user. In this implementation, the user can receive the digitally signed URI in the mail and use it to access information associated with the URI (e.g., by typing it into an internet web browser). In some implementations, the HCP sends the digitally signed URI as a piece of paper with a Quick Response (QR) code, which a user can scan or read when he or she receives it in the mail. The QR code can include a link address or URI related to the fitting data.

At verifying URI operation 325, the hearing device 102 or the electronic device 120 can verify the digitally signed URI 205. To verify the URI with the hearing device 102, the hearing device 102 can receive the digitally signed URI can determine whether it has been signing by the private key by using the public key (e.g., using a hash function, the digitally signed link, and the public key). The hearing device 102 can store the public key in its memory and use security algorithms to compute whether the digitally signed URI was signed. If the hearing device 102 determines that URI was digitally signed with the private key, the process 300 can continue to an establishing a session or sharing a communication operation 335. If the hearing device 102 determines that the URI was not properly signed, the process 300 can stop and the hearing device can notify a user that a URI is not proper or has not been properly signed or authenticated. The hearing device 102 can also determine whether a link has expired at the verifying URI operation 325.

To verify a digitally signed URI with the electronic device, the electronic device 120 or the hearing devices 102 should have the public key to determine if the private key was used to sign the URI. If the keys are asymmetrical (e.g., public, and private key), the electronic device 120 or the hearing devices 102 can use the public key to verify the URI is corrected. As described in Figure 1B, the verifying operation can include computing a hash function with the digitally signed link and the public key. If the output of the hash function is correct, it will correlate to an output that indicates the private key signed the URI. If the output is incorrect, it will indicate the link has been tampered with or the private key not used to sign the URI. The verifying URI operation 325 can also include determining whether the digitally signed URI has expired. If the URI is not valid or there is a security risk detected, the electronic device can notify the user with a warning (e.g., message).

In some implementations of the verifying URI operation 325, the hearing device 102 can use a symmetrical private key. For example, the HCP can use a symmetrical key stored on the hearing device to sign the link and can also use a symmetrical key to verify the link (e.g., another copy of the key). Here, the symmetrical key is a shared secret. Thus, if the symmetric key is not accessible untrusted parties, the URI is secure. To keep the symmetric key secret, the hearing device manufacture can store it in the hearing device or transmit it to only HCPs or hearing device users who have been verified (e.g., a secure email, identification checked, or Internet Protocol address verified).

At establishing session or sharing communication operation 335, the hearing device 102 or the electronic device 120 use the verified digitally signed URI to start a session or access information. For example, the verified URI can be an invitation to start a distance fitting session, where a user connects with an HCP over the Internet to share information related to the hearing device (e.g., fitting parameters). As another example, the verified URI can be an invitation to set up a fitting session such as a calendar invite to meet online or in person at a certain data and time. The verified URI can also provide a secure location on a cloud or network attached storage for the storing or access of hearing device data. For example, the verified URI can lead to a location on a storage cloud where the user can access or store personal information or information related to his or her hearing device. For example, the verified URI can be associated with fitting data for the hearing device or the user. In some implementations, the establishing a session or sharing communication operation 335 is optional in that the electronic device 120 stores the URI (e.g., for a later use) and does not immediately or shortly after receiving the URI execute an operation.

After the transmitting operation 330, the process 300 can stop, be repeated, or one or some of the operations can be repeated. In some implementations, the process 300 restarts if the HCP sends a new URI or wants to communicate with the hearing device user securely for during another session or regarding another matter. In some implementations, the process 300 occurs continuously while the HCP is sending URIs or information to an electronic device 120 or a user associated with a customer profile.

### Conclusion

Unless the context clearly requires otherwise, throughout the description and the claims, the words "comprise," "comprising," and the like are to be construed in an inclusive sense, as opposed to an exclusive or exhaustive sense; in the sense of "including, but not limited to." As used herein, the terms "connected," "coupled," or any variant thereof means any connection or coupling, either direct or indirect, between two or more elements; the coupling or connection between the elements can be physical, logical, electronic, magnetic, electromagnetic, or a combination thereof. Additionally, the words "above," and "below," and words of similar import, when used in this application, refer to this application and not to any portions of this application. Where the context permits, words in the above Detailed Description using the singular or plural number may also include the plural or singular number respectively. The word "or," in reference to a list of two or more items, covers all the following interpretations of the word: any of the items in the list, all the items in the list, any combination of the items in the list, or a single item from the list.

The teachings of the technology provided herein can be applied to other systems, not necessarily the system described above. The elements and acts of the various examples described above can be combined to provide further implementations of the technology. Some alternative implementations of the technology may include not only additional elements to those implementations noted above, but also may include fewer elements. For example, a user can use a hearing device to sign a URI that he or she created at his or her electronic device. In such an example, the user can use the private key stored on the hearing device and the HCP can have access to a public key.

The terms used in the following claims should not be construed to limit the technology to the specific examples disclosed in the specification, unless the above Detailed Description section explicitly defines such terms. Accordingly, the actual scope of the technology encompasses not only the disclosed examples, but also all equivalent ways of practicing or implementing the technology under the claims.

To reduce the number of claims, certain aspects of the technology are presented below in certain claim forms, but the applicant contemplates the various aspects of the technology in any number of claim forms. For example, while only one aspect of the technology is recited as a computer-readable medium claim, other aspects may likewise be embodied as a computer-readable medium claim, or in other forms, such as being embodied in a means-plus-function claim.

The techniques, algorithms, and operations introduced here can be embodied as special-purpose hardware (e.g., circuitry), as programmable circuitry appropriately programmed with software and/or computer code or computer code, or as a combination of special-purpose and programmable circuitry. Hence, embodiments may include a machine-readable medium having stored thereon instructions which may be used to program a computer (or other electronic devices) to perform a process. The machine-readable medium may include, but is not limited to, optical disks, compact disc read-only memories (CD-ROMs), magneto-optical disks, read-only memories (ROMs), random access memories (RAMs), erasable programmable read-only memories (EPROMs), electrically erasable programmable read-only memories (EEPROMs), magnetic or optical cards, flash memory, or other type of media such as machine-readable medium suitable for storing electronic instructions. The machine-readable medium includes non-transitory medium, where non-transitory excludes propagation signals or a computer-readable medium. For example, a processor can be connected to a non-transitory computer-readable medium that stores instructions for executing instructions by the processor such as instructions to generate, digitally sign, verify, or use a URI.

**Reference list**

| | |
|---|---|
| Communication environment | 100 |
| Hearing device or hearing devices | 102 |
| Processor | 103 |
| Antenna | 104 |
| Memory | 105 |
| Loudspeaker | 106 |
| Key Information | 107 |
| Security Algorithm | 108 |
| Fitting Station | 110 |
| Fitting Software | 115 |
| Electronic Device | 120 |
| Network | 125 |
| Hearing device manufacturer | 130 |
| Uniform Resource Indicator (URI) | 202 |
| Digitally Signed (URI) | 205 |
| Process | 300 |

## Claims

1. A method to communicate using a hearing device, wherein the method includes a hearing device (102) configured to digitally sign a uniform resource indicator (URI), a fitting station (110) configured to communicate with the hearing device (102), the method comprising:
generating, at the fitting station (110), a URI (202);
transmitting the URI (202) to the hearing device (102);
signing, at the hearing device (102), the URI (202) to generate a digitally signed URI (205) from the URI (202);
transmitting the digitally signed URI (205) from the fitting station (110) to an electronic device (120);
verifying the digitally signed URI (205) at the electronic device (120); and
based on verifying the digitally signed URI (205), using the digitally signed URI (205) to establish a session between the fitting station (110) and the hearing device (102) or using the digitally signed URI (205) to access a network or computer address.

2. The method of claim 1, wherein signing the URI (202) further comprises:
using a private key stored in a memory (105) for the hearing device (102), where the private key is associated with a public key and the electronic device (120) has access to the public key, is configured to receive access to the public key, or is configured to receive the public key.

3. The method of claim 2, wherein the private key and the public key are an asymmetric key pair.

4. The method of claim 2, the method further comprising:
distributing the public key over a network; and
wherein verifying the digitally signed URI (205) includes using the distributed public key at the electronic device (120) to verify the digitally URI (205).

5. The method of claim 2, wherein the private key or the public key was generated by the hearing device (102).

6. The method of claim 1, wherein the digitally signed URI is configured to expire.

7. The method of claim 2, wherein the private key or the public key was stored in a memory for the hearing device (102) by a hearing device manufacturer at the time of manufacture.

8. The method of claim 1, wherein the digitally signed URI (205) is associated with a fitting session, fitting data, a serial number for the hearing device, a user of the hearing device, or an invitation from a hearing care professional (HCP).

9. The method of claim 1, transmitting the URI (202) to the hearing device (102) is performed using a Bluetooth™ communication protocol.

10. The method of claim 1, wherein the electronic device (120) is a mobile device configured to use an application to use the digitally signed URI (205).

11. The method of claim 1, wherein the hearing device (102) is a hearing aid.

12. The method of claim 1, wherein the hearing device (102) is binaurally paired with another hearing device (102).

13. The method of claim 1, wherein digitally signing the URI (202) further comprises: using a symmetrical key to sign to the URI.

14. A computer-readable medium, in which a computer program according to any one of the preceding claims is stored.

15. A hearing device (102) configured to perform any of the method claims 1-13.
